# EUROPÄISCHE PATENTSCHRIFT

(11) **EP 2 594 494 B1**
(45) Veröffentlichungstag und Bekanntmachung des Hinweises auf die Patenterteilung: **12.04.2017**
(21) Anmeldenummer: 12193688.4
(22) Anmeldetag: 21.11.2012
(51) Int. Cl.: B65B 55/08, B65B 57/00, A61L 2/08, B67C 7/00

(54) **Vorrichtung und Verfahren zum Sterilisieren von Kunststoffbehältnissen mit Elektronenstrahlung**
Device and method for sterilising plastic containers with electron radiation
Dispositif et procédé de stérilisation de récipients en plastique avec rayonnement électronique

(30) Priorität: 21.11.2011 DE 102011055550
(43) Veröffentlichungstag der Anmeldung: 22.05.2013
(73) Patentinhaber: Krones AG, 93073 Neutraubling (DE)
(72) Erfinder: Frankenberger, Günter, 93073 Neutraubling (DE); Knott, Josef, 93073 Neutraubling (DE)
(74) Vertreter: Bittner, Bernhard

(56) Entgegenhaltungen:
- EP-A1- 2 141 073
- EP-A1- 2 295 324
- US-A1- 2011 012 032

## Beschreibung

Die vorliegende Erfindung bezieht sich auf eine Vorrichtung und ein Verfahren zum Sterilisieren von Behältnissen und insbesondere von Kunststoffbehältnissen. Aus dem Stand der Technik sind diverse Verfahren zum Sterilisieren von Behältnissen bekannt und insbesondere auch Verfahren, mit denen die Innenwandungen dieser Behältnisse sterilisiert werden können. Üblicherweise findet dabei eine Sterilisierung mittels chemischer Reinigungsmittel statt, wie beispielsweise Wasserstoffperoxid oder Peressigsäure.

In jüngerer Zeit ist man jedoch bestrebt, auf den Einsatz chemischer Reinigungsmittel möglichst weitgehend zu verzichten. Daher sind aus dem Stand der Technik auch Verfahren und Vorrichtungen bekannt geworden, welche eine Sterilisation in anderer Weise bewirken, beispielsweise durch eine Beaufschlagung der jeweiligen Behältnisoberflächen mittels Ladungsträgern.

So beschreibt beispielsweise die WO 2011/011079 A1 eine Vorrichtung zum Sterilisieren von Behältnissen mittels Elektronenstrahlung. Bei dieser Vorrichtung wird ein Stangenkörper, der an seinem unteren Ende ein Austrittsfenster für Elektronen aufweist, in das Behältnis eingeführt und die so austretenden Elektronen gelangen auf die Innenwandung der Behältnisse, um diese zu sterilisieren. Diese Vorrichtung weist auch eine Kontrolleinrichtung zum Erfassen eines Ausfalls der Elektronen auf, wobei nach dessen Erfassung die Betriebsgeschwindigkeit der Vorrichtung verringert wird, bis dieser Ausfall kompensiert ist.

Technisch bedingt treten jedoch bei derartigen Elektronenemittern von Zeit zu Zeit interne Überschläge, so genannte Sparks bzw. auch als arcs bezeichnet auf, welche dazu führen, dass die Emitterspannung kurzzeitig abgeschaltet werden muss. Die Spannung wird zwar innerhalb von Milli- bis Zentelsekunden wieder aufgebaut, innerhalb dieser Zeitspanne ist jedoch die austretende Elektronenstrahlung nicht bzw. nur eingeschränkt vorhanden. Wird ein derartiger Emitter zur Sterilisation, beispielsweise von Verpackungsmaterialien, eingesetzt, ist für die Sicherstellung der Entkeimung das Aufbringen einer konstanten Strahlungsdosis bzw. einer Mindestdosis unbedingt Voraussetzung. Dabei ist es einerseits erforderlich, die Behältnisse selbst zu Sterilisieren und andererseits auch, die Sterilität des Reinraums, in den die Behälter anschließend einfahren, nicht durch Keimverschleppung zu gefährden.

Bei statischer Bestrahlung kann dabei die Bestrahlungszeit entsprechend erhöht werden. Bei einem Einsatz in Maschinen, in denen die Packmittel kontinuierlich an den Emittern vorbeigeführt werden, (wie dies beispielsweise bei aus dem internen Stand der Technik der Anmelderin bekannten Füllmaschinen der Fall ist) ist die Verweilzeit wegen der Transportgeschwindigkeit stark eingeschränkt.

In der EP 2141073 A1 wird ein Lösungsansatz für dieses Problem beschrieben. Dabei wird die Behandlungsstrecke so lange gewählt, dass trotz derartiger Strahlungsaussetzer (im Folgenden als arcs bezeichnet) die für die Entkeimung notwendige Mindeststrahlung auf der Behälteroberfläche immer erreicht wird. Ein Nachteil ist, dass im "Normalfall" das Verpackungsmaterial immer überbestrahlt wird, was sich negativ auf das Material selbst auswirken kann, beispielsweise in Form von sogenanntem Yellowing oder einer Verlinkung von PET oder auch PE-Polymeren. Ein Erhöhen der Strahlungsleistung nach einem arc, um dadurch die fehlende Dosis nachzureichen, ist mit der momentan vorhandenen Regelungstechnik nur bedingt möglich und insbesondere dann nahezu unmöglich, wenn der besagte arc ganz am Ende der Behandlung des Behältnisses eintritt. Weiterhin ist eine derartige Vorgehensweise auch mit einer Anpassung der (Blei-) Abschirmung an die Maximalleistung des Emitters verbunden.

Der vorliegenden Erfindung liegt daher die Aufgabe zu Grunde, eine Vorrichtung und ein Verfahren zum Sterilisieren von Behältnissen mittels Ladungsträgern zu verwirklichen, welches auch bei Auftreten der oben genannten Aussetzer eine ausreichende Sterilisierung der Behältniswandungen gewährleistet.

Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältnissen mittels Ladungsträgern weist eine erste Transporteinrichtung auf, welche die Behältnisse entlang eines vorgegebenen Transportpfades transportiert. Weiterhin weist die Vorrichtung eine Vielzahl von an dieser Transporteinrichtung angeordneten Sterilisationseinrichtungen auf, wobei die Sterilisationseinrichtungen jeweils Ladungsträgererzeugungseinrichtungen zum Erzeugen der Ladungsträger sowie Beschleunigungseinrichtungen zum Beschleunigen der Ladungsträger und stangenartige Ladungsträgerleitungseinrichtungen aufweisen, wobei diese Ladungsträgerleitungseinrichtungen in die Behältnisse durch deren Mündungen einführbar sind. Weiterhin weisen die Ladungsträgerleitungseinrichtungen Austrittsfenster auf, durch welche die Ladungsträger aus den Ladungsträgerleitungseinrichtungen austreten.

Erfindungsgemäß weisen die einzelnen Sterilisationseinrichtungen jeweils Bewegungseinrichtungen auf, um die Behältnisse bezüglich der stangenartigen Ladungsträgerleitungseinrichtungen in einer Längsrichtung der Behältnisse zu bewegen, wobei die Bewegungsprofile der Bewegungen der Behältnisse gegenüber den diesen jeweils zugeordneten Ladungsträgerleitereinrichtungen unabhängig voneinander steuerbar sind und die Vorrichtung wenigstens eine Erfassungseinrichtung aufweist, welche einen temporären (insbesondere störungsbedingten) Ausfall der beschleunigten Ladungsträger der einzelnen Sterilisationseinrichtungen erfasst.

Dabei sind erfindungsgemäß die Bewegungseinrichtungen derart mit der oder den Erfassungseinrichtungen wirkverbunden und (insbesondere signaltechnisch) gekoppelt, dass bei Erfassen eines Ausfalls der beschleunigten Ladungsträger bei einer Sterilisationseinrichtung durch die Erfassungseinrichtung eine Relativbewegung zwischen dieser Sterilisationseinrichtung und dem von dieser Sterilisationseinrichtung zu sterilisierenden Behältnisses angehalten und/oder umgekehrt wird, wobei nach Wiederherstellung der Strahlerleistung weitergefahren wird.

Erfindungsgemäß werden bei dieser modifizierten Bewegung der Behältnisse in deren Längsrichtung gegenüber der Ladungsträgerleitungseinrichtung die Behältnisse weiterhin mit konstanter Transportgeschwindigkeit von der Transporteinrichtung entlang des Transportpfades transportiert.

Es werden damit die oben erwähnten arcs erfasst und diese werden durch eine entsprechende Anpassung des Bewegungsprofils kompensiert, wobei die Relativbewegung angehalten oder sogar umgekehrt wird. Vorteilhaft handelt es sich bei den Antriebseinrichtungen um elektromotorische Antriebe (z.B. Linearmotoren), wobei jedoch auch pneumatische, magnetische oder hydraulische Antriebe Anwendung finden können.

Weiterhin weist die Vorrichtung vorteilhaft eine Kühleinrichtung auf, welche die Austrittsfenster wenigstens zeitweise mit einem gasförmigen Medium beaufschlagt. Diese Kühlungseinrichtung kann dabei einen Kanal zum Leiten des gasförmigen Mediums aufweisen, der sich entlang der stangenartigen Ladungsträgerleitungseinrichtung erstreckt. Vorteilhaft ist die Transporteinrichtung derart ausgestaltet, dass sie die Behältnisse kontinuierlich transportiert. Besonders bevorzugt handelt es sich bei der Transporteinrichtung um ein drehbares Rad, an dem die einzelnen Sterilisationseinrichtungen angeordnet sind. Damit erfolgt bevorzugt der Transport der Behältnisse auf einer kreisförmigen Bahn.

Bei einer weiteren vorteilhaften Ausführungsform ist die Bewegung der stangenförmigen Ladungsträgerleitungseinrichtung gegenüber dem Behältnis an ein Profil dieses Behältnisses angepasst, sodass beispielsweise bei wechselnden Querschnitten dieses Behältnisprofils auch die Relativgeschwindigkeit zwischen dem Behältnis und der Ladungsträgerleitungseinrichtung bzw. deren Austrittsfenster variiert wird.

Bei einer weiteren vorteilhaften Ausführungsform weist die Vorrichtung Ablenkeinrichtungen auf, welche die Ladungsträger in einer senkrecht zu der Richtung der Relativbewegung stehenden Richtung ablenken, um diese so in Richtung der Innenwandung der Behältnisse zu lenken.

Es wird daher auch vorgeschlagen, dass bei den beschriebenen kontinuierlich laufenden Maschinen nicht die Behandlungsstrecke der notwendigen Bestrahlungsdosis angepasst wird, sondern die Behandlungszeit und dies durch die besagte Anpassung der Relativbewegung zwischen der Ladungsträgerleitungseinrichtung und den Behältnissen erfolgt. Dabei werden die normalerweise mit einem definierten Bewegungsprofil transportierten Packungsmittel bei Auftreten eines derartigen arcs sofort gestoppt und bei Bedarf über die dabei zurückgelegte Strecke zurück bewegt und nach Wiederherstellung der Strahlerleistung weiter gefahren. Vorteilhaft bewegen die Bewegungseinrichtungen die einzelnen Behältnisse relativ zu den Ladungsträgerleitungseinrichtungen, das heißt, dass bei dieser Ausgestaltung die Ladungsträgerleitungseinrichtungen selbst nicht in der Längsrichtung der Behältnisse bewegt werden. Es wäre jedoch auch möglich, umgekehrt die Ladungsträgerleitungseinrichtungen gegenüber den Behältnissen in deren Längsrichtung zu bewegen und die Behältnisse selbst nicht zu bewegen. Auch könnte eine Kombination aus diesen beiden Bewegungen erfolgen. So könnte beispielsweise im Rahmen des normalen Arbeitsbetriebs jeweils das Behältnis bewegt werden und im Falle des Auftretens von arcs zusätzlich die Ladungsträgerleitungseinrichtung und zwar derart, dass die besagte Kompensation des Ausfalls auftritt.

Zusätzlich oder anstelle der hier beschriebenen Maßnahme wäre es auch möglich, auf einen derartigen Ausfall der Ladungsträgerstrahlung mit einer modifizierten seitlichen Ablenkung der Ladungsträger zu reagieren. So könnte beispielsweise durch das gezielte Ansteuern von seitlichen Spulen nach dem Auftreten eines arcs die Ablenkung der Ladungsträger derart modifiziert werden, dass auch die nicht sterilisierten Bereiche noch mit diesem beaufschlagt werden.

Bevorzugt handelt es sich, wie oben erwähnt, bei den Ladungsträgern um Elektronen.

Bei einer vorteilhaften Ausführungsform steuert eine Steuerungseinrichtung die Sterilisationseinrichtungen derart, dass diese eine Ladungsträgerstrahlung zumindest dann ausgeben, wenn die Ladungsträgerleitungseinrichtungen aus den Behältnissen wieder herausgezogen werden. Dabei wird bei Erfassung eines Ausfalls der beschleunigten Ladungsträger die Relativbewegung der Ladungsträgerleitungseinrichtung gegenüber dem Behältnis angehalten wird oder die Ladungsträgerleitungseinrichtung zumindest abschnittsweise wieder in das Behältnis eingeführt.

Bei dieser Ausgestaltung wird daher vorgeschlagen, dass eine Sterilisation der besagten Behältnisinnenoberflächen insbesondere während einer Rückwärtsbewegung, das heißt, während des Herausziehens der Strahlfinger aus den Behältnissen stattfindet. Insbesondere diese Rückwärtsbewegung ist für die Sterilisation besonders effektiv, da hier die Sterilisation vom Boden des Behältnisses her stattfindet. So kann auch kurz nach dem Herausziehen des Strahlfingers aus dem Behältnis über dessen Mündung eine Ladungstragwolke erzielt werden, welche das Eintreten von Keimen in das Behältnis verhindert. Vorteilhaft bewegen die Bewegungseinrichtungen die Ladungsträgerleitungseinrichtungen schneller in die Behältnisse hinein als wieder aus diesen heraus, sodass der wesentliche Anteil des Sterilisierens während der Rückwärtsbewegung, d.h. während des Herausziehens der Ladungsträgerleitungseinrichtungen aus den Behältnissen erfolgt.

So ist es möglich, dass die einzelnen Ladungsträgerleitungseinrichtungen, im Folgenden auch als Strahlfinger bezeichnet, auf einem Karussell angeordnet sind. Über einen Einlauf- und Auslaufstern werden die zu behandelnden Behältnisse in das Karussell ein- und ausgefahren. In dem Karussell werden vorzugsweise die Behältnisse zentral unter den jeweiligen Strahlungsfingern positioniert. Gemäß eines der Strahlungsleistung und der Flaschengeometrie vorgegebenen Verfahrprofils fährt das Behältnis nun nach oben, der Strahlungsfinger taucht in das Behältnis ein und anschließend fährt das Behältnis wieder nach unten. Dabei wird, wie oben erwähnt, die Hubbewegung des Behältnisses über einen elektrischen Antrieb gesteuert und ist vorteilhaft mit dem Regelkreis des Strahlungsemitters verbunden. Im Falle eines arcs stoppt der Antrieb sofort die Bewegung des Behältnisses, bis die Spannung wieder hergestellt ist.

Bei einer weiteren vorteilhaften Ausführungsform weist daher die Vorrichtung eine zweite Transporteinrichtung auf, welche die Behältnisse von der ersten Transporteinrichtung an einer vorgegebenen Übernahmeposition übernimmt. Weiterhin ist vorteilhaft eine weitere Transporteinrichtung, insbesondere in Form eines Transportsterns, vorgesehen, welche die Behältnisse an die oben beschriebene Vorrichtung zum Sterilisieren der Behältnisse übergibt. Bei einer weiteren vorteilhaften Ausführungsform ist eine Zeitspanne, während derer sich das Austrittsfenster innerhalb der Behältnisse befindet, veränderbar. Damit ist vorteilhaft auch ein Prozesswinkel, entlang dessen die Behältnisse insgesamt sterilisiert werden, veränderbar.

Vorteilhaft ist damit nach dem Herausziehen der Strahlungsfinger aus den Behältnissen eine variable Transportstrecke der Behältnisse bis zur Übergabe an die weitere Transporteinrichtung vorgesehen.

Bei einer weiteren vorteilhaften Ausführungsform weist die Vorrichtung einen Reinraum auf, innerhalb dessen die Kunststoffbehältnisse transportiert werden. Dabei findet also die Sterilisation der Behältnisse mit den Ladungsträgern insbesondere während deren Transport durch den besagten Reinraum statt. Bei einer vorteilhaften Ausführungsform wird dieser Reinraum auch abschnittsweise durch die besagte Transporteinrichtung ausgebildet. So kann beispielsweise die Transporteinrichtung als Rad ausgebildet sein, wobei beispielsweise eine Wandung dieses Rades gleichzeitig auch eine Begrenzungswandung für den besagten Reinraum darstellt.

Wie oben erwähnt, ist vorteilhaft die Transporteinrichtung als drehbarer Träger ausgestaltet.

Die vorliegende Erfindung ist weiterhin auf ein Verfahren zum Sterilisieren von Behältnissen gerichtet, wobei die Behältnisse mit einer Transporteinrichtung entlang eines vorgegebenen Transportpfades transportiert werden und während dieses Transports zumindest eine Innenwandung der Behältnisse durch Beaufschlagung mit Ladungsträgern sterilisiert wird. Dabei werden zur Sterilisation der Behältnisse Ladungsträger erzeugt und mittels einer Beschleunigungseinrichtung innerhalb einer stangenförmigen Ladungsträgerleiteinrichtung beschleunigt, wobei die Ladungsträgerleitungseinrichtung mit einem vorgegebenen Bewegungsprofil durch eine Relativbewegung zwischen der Ladungsträgerleitungseinrichtung und dem Behältnis entlang einer Längsrichtung des Behältnisses in das Behältnis eingeführt wird und anschließend aus dem Behältnis ausgeführt wird und die Innenwandung des Behältnisses mit den Ladungsträgern beaufschlagt wird, während sich die Ladungsträgerleitungseinrichtung zumindest abschnittsweise innerhalb des Behältnisses befindet.

Erfindungsgemäß erfasst eine Erfassungseinrichtung einen temporären Ausfall der Ladungsträgerstrahlung und bewirkt in Reaktion auf einen Ausfall der Ladungsträgerstrahlung, dass eine Relativbewegung zwischen dem Behältnis und der Ladungsträgerleitungseinrichtung angehalten und/oder in ihrer Bewegungsrichtung umgekehrt wird.

Vorteilhaft werden die Behältnisse kontinuierlich entlang ihres Transportpfades transportiert.

Bei einem bevorzugten Verfahren erfolgt eine Sterilisation der Behältnisse, während die Ladungsträgerleitungseinrichtung aus den Behältnissen herausgezogen wird. Im Rahmen von Entwicklungsarbeiten wurde seitens der Anmelderin ermittelt, dass insbesondere während dieses Teilprozesses des Herausziehens des Strahlfingers in besonders vorteilhafter Weise eine Sterilisation der Innenwandung der Behältnisse erreicht werden kann. Damit werden bei diesem Verfahren die Behältnisse "von unten her", das heißt, von ihrem Boden her, sterilisiert.

Bei einem weiteren vorteilhaften Verfahren wird die Zeitspanne, während derer sich die Ladungsträgerleitungseinrichtungen in den Behältnissen befindet, bei Auftreten von Ausfällen der Ladungsträgerstrahlung verlängert.

Es wird darauf hingewiesen, dass die vorliegende Erfindung auch auf die Sterilisation die Sterilisation von anderen Behältniskomponenten und insbesondere auf die Sterilisation von Behältnisaußenwandungen und Behältnisverschlüssen anwendbar ist.

Eine derartige Vorrichtung zum Sterilisieren von Behältniskomponenten und insbesondere von Behältnisaußenwandungen und/oder Behältnisverschlüssen mittels Ladungsträgern weist eine erste Transporteinrichtung auf, welche die Behältniskomponenten entlang eines vorgegebenen Transportpfades transportiert, wenigstens eine insbesondere gegebnüber dem Transportpfad stationär angeordnete Sterilisationseinrichtung wobei die Sterilisationseinrichtung eine Ladungsträgererzeugungseinrichtung zum Erzeugen der Ladungsträger, eine Beschleunigungseinrichtung zum Beschleunigen der Ladungsträger, und ein Autrittsfenster aufweist durch welches die Ladungsträger austreten und auf die Behältniskomponenten richtbar sind.

Dabei weist die Vorrichtung wenigstens eine Erfassungseinrichtung auf, welche einen Ausfall der beschleunigten Ladungsträger der Sterilisationseinrichtung erfasst, wobei die Transporteinrichtung derart mit der Erfassungseinrichtung gekoppelt ist, dass bei Erfassen eines Ausfalls der beschleunigten Ladungsträger durch die Erfassungseinrichtung eine Transportbewegung der Behältnisse angehalten und/oder umgekehrt wird. Dabei wäre es möglich und auch bevorzugt, dass die Transporteinrichtung, welche die Behältnisse transportiert, selbst anhält oder ihre Bewegungsrichtung umkehrt. Es wäre jedoch auch möglich, über zusätzliche Mittel wie Behältnis- oder Verschlusssperren die Bewegung der Behältnisse anzuhalten oder auch umzukehren.

Bei einem entsprechenden Verfahren zum Sterilisieren von Behältniskomponenten und insbesondere von Behältnisaussenwandungen und/oder Behältnisverschlüssen werden die Behältniskomponenten mit einer Transporteinrichtung entlang eines vorgegebenen Transportpfades transportiert und während dieses Transports wird zumindest ein Bereich der Behältniskomponenten durch Beaufschlagung mit Ladungsträgern sterilisiert, wobei zur Sterilisation der Behältniskomponenten Ladungsträger erzeugt werden und mittels einer Beschleunigungseinrichtung beschleunigt werden. Erfindungsgemäß erfasst eine Erfassungseinrichtung einen temporären Ausfall der Ladungsträgerstrahlung und in Reaktion auf einen Ausfall der Ladungsträgerstrahlung wird eine Relativbewegung zwischen dem Behältnis und der Ladungsträgerleistungseinrichtung angehalten und/oder in der Bewegungsrichtung umgekehrt, wobei nach Wiederherstellung der Strahlerleistung weiter gefahren wird, wobei bei dieser modifizierten Bewegung der Behältnisse in deren Längsrichtung gegenüber der Ladungsträgerleitungseinrichtung die Behältnisse weiterhin mit konstanter Transportgeschwindigkeit von der Transporteinrichtung entlang des Transportpfades transportiert werden.

Es wird also auch in Bezug auf andere Behältniskomponenten vorgeschlagen, dass ein Ausfall der Ladungsträgerstrahlung durch ein Anhalten oder zumindest kurzzeitiges Umkehren der Transportbewegung, allerdings hier insbesondere der Transportbewegung der Behältnisse entlang ihres Transportpfads, kompensiert wird.

Dabei können beispielsweise Verschlüsse aktiv unter oder zwischen Strahlungseinrichtungen, bei denen es sich beispielsweise um Flächenemitter handeln kann, vorbeibewegt. Vorteilhaft sind dabei diese Flächenemitter für eine ausreichende Dosis für die kontinuierlich vorbeitransportierten Behältniskomponenten z.B. Verschlüsse ausgelegt.

Im Falle einer Sterilisation von Behältnissen, insbesondere der Aussenwandungen der Behältnisse können diese beispielsweise mittels eines Teilungsverzugssterns an den Sterilisationseinrichtungen, bei denen es sich auch hier bevorzugt um Flächenemitter handelt, vorbeitransportiert werden. Auch hier erfolgt der Transport der Behältnisse vorteilhaft kontinuierlich. Es wäre jedoch auch möglich, dass die Behältnisse entlang ihres Transportpfades getaktet transportiert werden.

Im Falle des Auftretens eines Arcs reagiert der Teilungsverzugsstern sofort und schwenkt die Behältnisse vorteilhaft entgegen der Laufrichtung bzw. entgegen der Transportrichtung nach hinten bzw. läßt das oder die betreffenden Behältnisse kurz stehen.

Wie oben erwähnt, handelt es sich bei den zu sterilisierenden Behältnissen insbesondere um Flaschen, es wäre jedoch auch möglich, die Erfindung auf die Sterilisation von Kunststoffvorformlingen, welche in Kunststoffbehältnisse umformbar sind, anzuwenden.

Weitere Vorteile und Ausführungsformen ergeben sich aus den beigefügten Zeichnungen: Darin zeigen:
- Fig. 1: eine schematische Darstellung einer erfindungsgemäßen Vorrichtung;
- Fig. 2: eine schematische Darstellung einer Sterilisationseinrichtung; und
- Fig. 3a-3c: drei Darstellungen für einen Sterilisationsprozess.

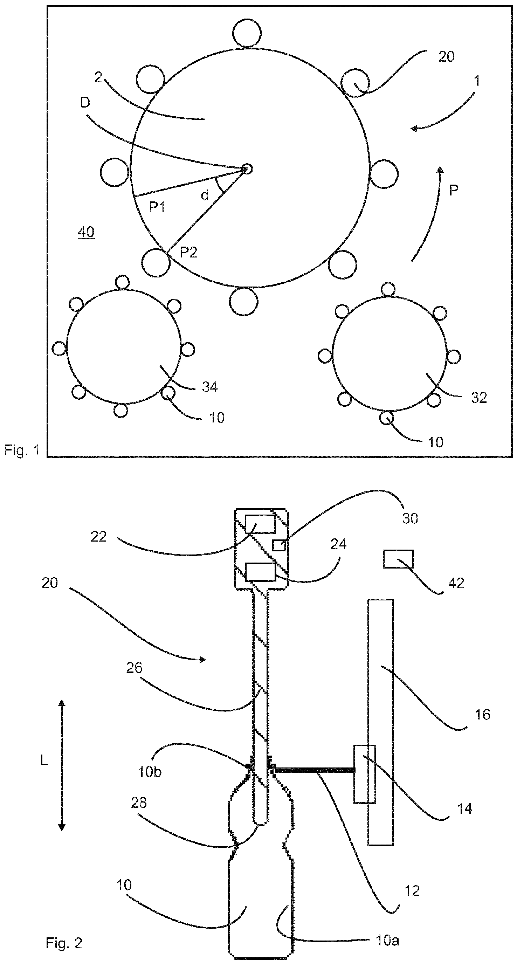

Fig. 1 zeigt eine grob schematische Darstellung einer erfindungsgemäßen Vorrichtung 1. Diese Vorrichtung weist eine Transporteinrichtung 32 wie einen Zuführstern auf, welcher der Transporteinrichtung 2, bei der es sich insbesondere um ein um eine Drehachse D drehbares Rad handelt, die zu sterilisierenden Behältnisse 10 zuführt. Das Bezugszeichen 34 kennzeichnet eine weitere Transporteinrichtung 34, welche vorteilhaft ebenfalls als Transportstern ausgeführt ist und welche die sterilisierten Behältnisse 10 von der Transporteinrichtung abfördert. Der Umfangswinkel, unter dem die Behältnisse 10 zugeführt werden und auch der Umfangswinkel, unter dem die Behältnisse 10 abgeführt werden, sind damit festgelegt. Das Bezugszeichen P kennzeichnet den Transportpfad der Behältnisse 10 während deren Sterilisation.

Das Bezugszeichen P1 kennzeichnet die Position, an der das die Ladungsträgerleitungseinrichtung wieder vollständig aus dem Behältnis heraus gezogen ist und das Bezugszeichen P2 kennzeichnet die Übergabeposition, an der das sterilisierte Behältnis abgegeben wird. Das Bezugszeichen d kennzeichnet einen Umfangswinkel, während dessen im normalen Arbeitsbetrieb die Ladungsträgerleitungseinrichtung 26 zwar bereits wieder aus dem Behältnis 10 herausgezogen ist, jedoch das Behältnis 10 noch von der Transporteinrichtung 2 transportiert wird. Bei Auftreten von Ausfällen kann dieser Umfangswinkel d verkürzt werden, so dass auch bei einer Verzögerung noch genügend Zeit bleibt, um die Ladungsträgerleitungseinrichtung 26 aus dem Behältnis 10 abzuziehen.

An der Transporteinrichtung 2 ist eine Vielzahl von Sterilisationseinrichtungen 20 angeordnet, welche ebenfalls entlang des Transportpfades P transportiert werden und während dieses Transports die Behältnisse 10 an deren Innenwandungen sterilisieren. Weiterhin weist die Vorrichtung 1 einen Reinraum 40 auf, innerhalb dessen die Sterilisation der Behältnisse 10 stattfindet. Dieser Reinraum kann dabei, wie in Fig. 1 gezeigt, durch ein die gesamte Anlage umgebendes Gehäuse ausgebildet sein, es wäre jedoch auch möglich, dass der Reinraum den Transportpfad P der Behältnisse 10 kanalartig umgibt.

Fig. 2 zeigt eine schematische Darstellung einer Sterilisationseinrichtung 20. Diese Sterilisationseinrichtung 20 weist eine Ladungsträgererzeugungseinrichtung 22 auf, welche Ladungsträger und insbesondere Elektronen erzeugt. Diese Ladungsträger werden mittels einer Beschleunigungseinrichtung 24 in Richtung eines Austrittsfensters 28 beschleunigt, wobei dieses Austrittsfenster 28 hier an einem unteren Ende einer stangenartigen Ladungsträgerleitungseinrichtung 26 angeordnet ist.

Bei diesem Austrittsfenster 28 kann es sich beispielsweise um ein Titanfenster handeln, durch welches hindurch die Elektronen als Elektronenwolke austreten können. Das Bezugszeichen 30 kennzeichnet grob schematisch eine Erfassungseinrichtung, welche zum Erfassen von arcs bzw. von Ausfällen der Ladungsträgerstrahlung dient. Dabei ist es möglich, dass diese Erfassungseinrichtung auch die Zeitspanne erfasst, während derer ein arc bzw. Ausfall aufgetreten ist. Daneben ist es auch möglich, dass das Auftreten eines derartigen arcs - beispielsweise in der Maschinensteuerung - protokolliert wird.

Das Bezugszeichen 12 bezieht sich auf eine Halteeinrichtung, welche die Behältnisse 10 greift, beispielsweise unterhalb deren Tragring. Diese Halteeinrichtung 12 ist dabei an einer Bewegungseinrichtung 14 angeordnet, welche die Behältnisse 10 entlang deren Längsrichtung L bewegt. Durch diese Bewegung wird erreicht, dass die Ladungsträgerleitungseinrichtung 26 und damit auch das Austrittsfenster 28 mehr oder weniger tief in das Behältnis 10 eintauchen. Die aus dem Austrittsfenster 28 austretenden Elektronen können somit auf die Innenwandung 10a des Behältnisses treffen und diese sterilisieren. Das Bezugszeichen 16 kennzeichnet einen Träger, an dem die Bewegungseinrichtung angeordnet ist. Bei der Bewegungseinrichtung kann es sich beispielsweise um einen Linearmotor handeln.

Das Bezugszeichen 42 bezieht sich auf eine Steuerungseinrichtung, welche die Bewegungseinrichtung 14 und damit die Bewegung der Kunststoffbehältnisse 10 in der Längsrichtung L steuert. Diese Steuerungseinrichtung 42 steht dabei auch in Signalverbindung mit der Erfassungseinrichtung 30 und kann so die Bewegung der Behältnisse in Reaktion auf das Feststellen eines Ausfalls der Elektronenstrahlung beeinflussen, insbesondere also anhalten oder umkehren.

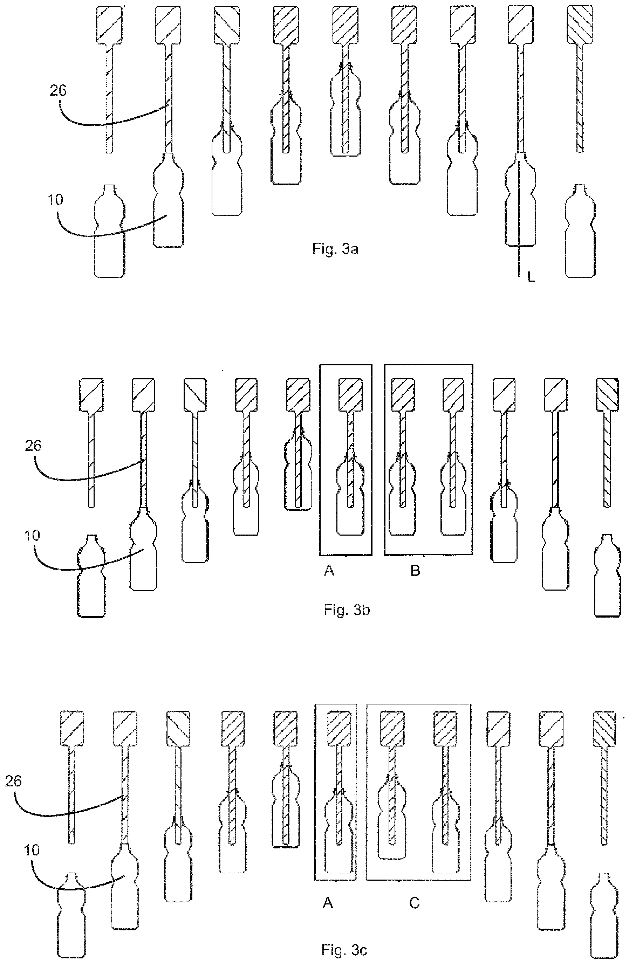

Die Figuren 3a - 3c veranschaulichen einen erfindungsgemäßen Sterilisationsprozess. In Fig. 3a ist dabei ein gewöhnlicher Ablauf veranschaulicht, d.h., ein Ablauf, bei dem keine Ausfälle der Sterilisationseinrichtungen auftreten. Hier wird das Behältnis 10 zunächst angehoben und so die Ladungsträgerleitungseinrichtung 26 durch dessen Mündung 10b hindurch in dessen Inneres eingetaucht. Anschließend wird die Ladungsträgerleitungseinrichtung wieder aus dem Behältnis 10 herausgezogen. Insbesondere dieser Prozess des Herausziehens ist dabei für den Sterilisationsvorgang von besonderer Bedeutung.

Bei der in Fig. 3b gezeigten Situation wurde zu dem Zeitpunkt A das Auftreten eines arcs festgestellt. In Reaktion hierauf wird die Bewegung der Behältnisse 10 in der Längsrichtung L angehalten und das Austrittsfenster 28 verbleibt damit für eine vorgegebene Zeit gegenüber dem Behältnis auf einer vorgegebenen Höhe in der Richtung L (Zeitspanne B). Dabei ist es möglich, dass die Bewegung des Behältnisses 10 solange angehalten wird, bis die Funktion wieder voll hergestellt ist, insbesondere also, bis wieder Elektronen in ausreichender Intensität vorhanden sind. Es wäre jedoch auch möglich, dass die Stillstandsphase hierüber hinaus etwas länger gestaltet wird, was insbesondere auch von der für die Sterilisation der Behältnisse nötigen Strahlungsdosis abhängen kann.

Bevorzugt steuert die Steuerungseinrichtung die Bewegungseinrichtung 14 auch derart, dass nach dem Beenden des Stillstands das Behältnis 10 schneller (hier nach unten) bewegt wird, damit bei Erreichen des Übergabeumfangswinkels die Ladungsträgerleitungseinrichtung vollständig aus dem Behältnis herausgezogen ist und damit das Behältnis entfernt werden kann. Während dieser modifizierten Bewegung der Behältnisse in deren Längsrichtung L (gegenüber der Ladungsträgerleitungseinrichtung 26) werden jedoch die Behältnisse 10 weiterhin (erfindungsgemäß mit konstanter Transportgeschwindigkeit) von der Transporteinrichtung entlang des Transportpfades transportiert. Bevorzugt sind daher die Bewegung der Behältnisse 10 entlang ihres Transportpfads P einerseits und die Relativbewegung der Behältnisse gegenüber den diesen zugeordneten Sterilisationseinrichtungen andererseits voneinander entkoppelt.

Fig. 3c zeigt eine weitere mögliche Vorgehensweise bei Auftreten eines Ausfalls der Ladungsträger. Hier wird das Behältnis während der Zeitspanne C, welche auf das Auftreten eines Ausfalls zum Zeitpunkt A folgt, wieder geringfügig angehoben und daher die Ladungsträgerleitungseinrichtung wieder tiefer in das Behältnis eingeschoben. Anschließend wird das Behältnis wieder nach unten abgezogen.

Es wäre auch möglich, dass die beiden in den Fig. 3b und 3c gezeigten Vorgehensweisen kombiniert werden, etwa zunächst die Relativbewegung in der Längsrichtung L angehalten und anschließend umgekehrt wird. Es wäre jedoch auch möglich, dass die Reaktion auf einen Ausfall der Elektronenstrahlung auch in Abhängigkeit von einer Relativposition des Behältnisses gegenüber der Ladungsträgerleitungseinrichtung zum Zeitpunkt des Ausfalls ausgewählt wird. So kann es beispielsweise ausreichend sein, dass kurz vor dem Herausziehen der Ladungsträgerleitungseinrichtung aus dem Behältnis ein kurzer Stillstand der Behältnisbewegung in deren Längsrichtung L ausreichend sein kann, um für eine ausreichende Strahlungsdosis zu sorgen.

### Bezugszeichenliste

- 1: Vorrichtung
- 2: Transporteinrichtung
- 10: Behältnis
- 10a: Innenwandung des Behältnisses
- 10b: Mündung des Behältnisses
- 12: Halteeinrichtung
- 14: Bewegungseinrichtung
- 16: Träger
- 20: Sterilisationseinrichtung
- 22: Ladungsträgererzeugungseinrichtung
- 24: Beschleunigungseinrichtung
- 26: Ladungsträgerleitungseinrichtung
- 28: Austrittsfenster
- 30: Erfassungseinrichtung
- 32: Transporteinrichtung (Einlaufstern)
- 34: Transporteinrichtung (Auslaufstern)
- 40: Reinraum
- 42: Steuerungseinrichtung

- P: Transportpfad
- L: Längsrichtung
- A,B,C: Zeitpunkte, Zeitspannen
- d: Umfangswinkel
- P1, P2: Position

## Patentansprüche

1. Vorrichtung (1) zum Sterilisieren von Behältnissen (10) mittels Ladungsträgern mit einer ersten Transporteinrichtung (2), welche die Behältnisse (10) entlang eines vorgegebenen Transportpfades (P) transportiert, mit einer Vielzahl von Sterilisationseinrichtungen (20), welche an dieser Transporteinrichtung (2) angeordnet sind, wobei die Sterilisationseinrichtungen (20) jeweils Ladungsträgererzeugungseinrichtungen (22) zum Erzeugen der Ladungsträger, Beschleunigungseinrichtungen (24) zum Beschleunigen der Ladungsträger, und stangenartige Ladungsträgerleitungseinrichtungen (26), welche in die Behältnisse (10) durch deren Mündungen einführbar sind, aufweisen und wobei weiterhin die Ladungsträgerleitungseinrichtungen (26) Austrittsfenster (28) aufweisen, durch welche die Ladungsträger aus den Ladungsträgerleitungseinrichtungen (26) austreten,
wobei die Vorrichtung (1) wenigstens eine Erfassungseinrichtung (30) aufweist, welche einen Ausfall der beschleunigten Ladungsträger der einzelnen Sterilisationseinrichtungen (20) erfasst, **dadurch gekennzeichnet, dass** die einzelnen Sterilisationseinrichtungen (20) jeweils Bewegungseinrichtungen (14) aufweisen, um die Behältnisse (10) bezüglich der stangenartigen Ladungsträgerleitungseinrichtungen (26) in einer Längsrichtung (L) der Behältnisse (10) zu bewegen, wobei die Bewegungsprofile der Bewegungen der Behältnisse (10) gegenüber den diesen jeweils zugeordneten Ladungsträgerleitungseinrichtungen (26) unabhängig voneinander steuerbar sind, wobei die Bewegungseinrichtungen (14) derart mit der Erfassungseinrichtung (30) signalverbunden ist, dass bei Erfassen eines Ausfalls der beschleunigten Ladungsträger bei einer Sterilisationseinrichtung (20) durch die Erfassungseinrichtung (30) eine Relativbewegung zwischen dieser Sterilsationseinrichtung (20) und dem von dieser Sterilisationseinrichtung (20) zu sterilisierenden Behältnis 10 angehalten und/oder umgekehrt wird,
wobei nach Wiederherstellung der Strahlerleistung weitergefahren wird,
wobei bei dieser modifizierten Bewegung der Behältnisse in deren Längsrichtung (L) gegenüber der Ladungsträgerleitungseinrichtung (26) die Behältnisse (10) weiterhin mit konstanter Transportgeschwindigkeit von der Transporteinrichtung (2) entlang des Transportpfades (P) transportiert werden.

2. Vorrichtung (1) nach Anspruch 1,
**dadurch gekennzeichnet, dass**
eine Steuerungseinrichtung (42) die Sterilisationseinrichtungen (20) derart steuert, dass diese eine Ladungssträgerstrahlung zumindest dann ausgeben, während die Ladungsträgerleitungseinrichtungen (26) aus den Behältnissen (10) herausgezogen werden und bei Erfassung eines Ausfalls der beschleunigten Ladungsträger die Relativbewegung der Ladungsträgerleitungseinrichtung (26) gegenüber dem Behältnis (10) angehalten wird, und/oder die Ladungsträgerleitungseinrichtung (26) zumindest abschnittsweise wieder in das Behältnis 10 eingeführt wird.

3. Vorrichtung (1) nach Anspruch 1,
**dadurch gekennzeichnet, dass**
die Vorrichtung (1) eine zweite Transporteinrichtung (34) aufweist, welche die Behältnisse (10) von der ersten Transporteinrichtung (2) an einer vorgegebenen Übernahmeposition (P2) übernimmt.

4. Vorrichtung (1) nach Anspruch 3,
**dadurch gekennzeichnet, dass**
eine Zeitspanne, während derer sich das Austrittsfenster innerhalb der Behältnisse (10) befindet, veränderbar ist.

5. Vorrichtung (1) nach wenigstens einem der vorangegangenen Ansprüche,
**dadurch gekennzeichnet, dass**
die Vorrichtung (1) einen Reinraum (40) aufweist, innerhalb dessen die Kunststoffbehältnisse (10) transportiert werden.

6. Vorrichtung (1) nach wenigstens einem der vorangegangenen Ansprüche,
**dadurch gekennzeichnet, dass**
die Transporteinrichtung (2) ein drehbarer Träger (2) ist.

7. Verfahren zum Sterilisieren von Behältnissen (10), wobei die Behältnisse (10) mit einer Transporteinrichtung (2) entlang eines vorgegebenen Transportpfades (P) transportiert werden und während dieses Transports zumindest eine Innenwandung (10a) der Behältnisse (10) durch Beaufschlagung mit Ladungsträgern sterilisiert wird, wobei zur Sterilisation der Behältnisse (10) Ladungsträger erzeugt werden und mittels einer Beschleunigungseinrichtung (24) innerhalb einer stangenförmigen Ladungsträgerleitungseinrichtung (26) beschleunigt werden, wobei die Ladungsträgerleitungseinrichtung (26) mit einem vorgegebenen Bewegungsprofil durch eine Relativbewegung zwischen der Ladungsträgerleitungseinrichtung (26) und dem Behältnis (10) entlang einer Längsrichtung (L) des Behältnisses (10) in das Behältnis (10) eingeführt wird, und anschließend aus dem Behältnis (10) ausgeführt wird, und die Innenwandung des Behältnisses (10) mit den Ladungsträgern beaufschlagt wird, während sich die Ladungsträgerleitungseinrichtung (26) zumindest abschnittsweise innerhalb des Behältnisses (10) befindet , wobei eine Erfassungseinrichtung (30) einen temporären Ausfall der Ladungsträgerstrahlung erfasst, **dadurch gekennzeichnet, dass** die Erfassungseinrichtung (30) in Reaktion auf einen Ausfall der Ladungsträgerstrahlung eine Relativbewegung zwischen dem Behältnis (10) und der Ladungsträgerleitungseinrichtung anhält und/oder in der Bewegungsrichtung umkehrt, wobei nach Wiederherstellung der Strahlerleistung weitergefahren wird,
wobei bei dieser modifizierten Bewegung der Behältnisse in deren Längsrichtung (L) gegenüber der Ladungsträgerleitungseinrichtung (26) die Behältnisse (10) weiterhin mit konstanter Transportgeschwindigkeit von der Transporteinrichtung (2) entlang des Transportpfades (P) transportiert werden.

8. Verfahren nach Anspruch 7,
**dadurch gekennzeichnet, dass**
eine Sterilisation der Behältnisse (10) erfolgt, während die Ladungsträgerleitungseinrichtung (26) aus den Behältnissen (10) herausgezogen wird.

9. Verfahren nach Anspruch 7,
**dadurch gekennzeichnet, dass**
die Zeitspanne, während der sich die Ladungsträgerleitungseinrichtungen (26) in den Behältnissen (10) befinden, bei Auftreten von Ausfällen verlängert wird.

10. Verfahren nach Anspruch 7,
**dadurch gekennzeichnet, dass**
die Bewegung der Behältnisse (10) entlang ihres Transportpfads (P) einerseits und die Relativbewegung der Behältnisse (10) gegenüber den diesen zugeordneten Sterilisationseinrichtungen (20) andererseits voneinander entkoppelt sind.

## Claims

1. An apparatus (1) for the sterilization of containers (10) by means of charge carriers with a first conveying device (2) which conveys the containers (10) along a pre-set conveying path (P), with a plurality of sterilization devices (20) which are arranged on this conveying device (2), wherein the sterilization devices (20) have in each case charge carrier production devices (22) for producing the charge carriers, acceleration devices (24) for accelerating the charge carriers and rod-like charge carrier guiding devices (26), which are capable of being introduced into the containers (10) through the apertures thereof, wherein, in addition, the charge carrier guiding devices (26) have outlet windows (28) through which the charge carriers issue out of the charge carrier guiding devices (26), wherein the apparatus (1) has at least one detection device (30) which detects the failure of the accelerated charge carriers of the individual sterilization devices (20),
**characterized in that**
the individual sterilization devices (20) have in each case movement devices (14) in order to move the containers (10) in a longitudinal direction (L) of the containers (10) with respect to the rod like charge carrier guiding devices (26), wherein the profiles of the movements of the containers (10) are controllable independently of one another with respect to the charge carrier guiding devices (26) associated with the aforesaid containers (10) in each case, wherein the movement devices (14) are connected by means of signals to the detection device (30) in such a way that, if a failure of the accelerated charge carriers is detected in a sterilization device (20) by the detection device (30), a relative movement between this sterilization device (20) and the container 10 to be sterilized by this sterilization device (20) is stopped and/or reversed, wherein after the restoration of the radiator power the movement is continued, wherein with this modified movement of the containers in their longitudinal direction (L) compared with the charge carrier guiding device (26) the containers (10) are furthermore conveyed with a steady conveying speed by the conveying device (2) along the conveying path (P).

2. An apparatus (1) according to claim 1,
**characterized in that**
a control device (42) controls the sterilization devices (20) in such a way that they emit a charge carrier radiation at least when the charge carrier guiding devices (26) are withdrawal from the containers (10), and, if a failure of the accelerated charge carriers is detected, the relative movement of the charge carrier guiding device (26) with respect to the container (10) is stopped, and/or the charge carrier guiding device (26) is introduced into the container (10) again at least locally.

3. An apparatus (1) according to claim 1,
**characterized in that**
the apparatus (1) has a second conveying device (34) which transfers the containers (10) from the first conveying device (2) to a pre-set transfer position (P2).

4. An apparatus (1) according to claim 3,
**characterized in that**
a period of time during which the outlet window is situated inside the containers (10) is variable.

5. An apparatus (1) according to at least one of the preceding claims,
**characterized in that**
the apparatus (1) has a clean room (40) inside which the plastic material containers (10) are conveyed.

6. An apparatus (1) according to at least one of the preceding claims,
**characterized in that**
the conveying device (2) is a rotatable carrier (2).

7. A method of sterilizing containers (10), wherein the containers (10) are conveyed by a conveying device (2) along a pre-set conveying path (P) and during this conveying at least one inner wall (10a) of the containers (10) is sterilized by being acted upon with charge carriers, wherein charge carriers are produced for the sterilization of the containers (10) and they are accelerated inside a rod-shaped charge carrier guiding device (26) by means of an acceleration device (24), wherein the charge carrier guiding device (26) is introduced with a pre-set movement profile into the container (10) by a relative movement between the charge carrier guiding device (26) and the container (10) along a longitudinal direction (L) of the container (10), and is then removed from the container (10) and the inner wall of the container (10) is acted upon with the charge carriers, while the charge carrier guiding device (26) is situated at least locally inside the container (10), wherein a detection device (30) detects a temporary failure of the charge carrier radiation,
**characterized in that**
the detection device (30) stops a relative movement between the container (10) and the charge carrier guiding device and/or reverses it in the direction of movement in reaction to a failure of the charge carrier radiation wherein after the restoration of the radiator power the movement is continued, wherein with this modified movement of the containers in their longitudinal direction (L) compared with the charge carrier guiding device (26) the containers (10) are furthermore conveyed with a steady conveying speed by the conveying device (2) along the conveying path (P).

8. A method according to claim 7,
**characterized in that**
a sterilization of the containers (10) is carried out while the charge carrier guiding device (26) is withdrawn out of the containers (10).

9. A method according to claim 7,
**characterized in that**
the period of time during which the charge carrier guiding devices (26) are present in the containers (10) is prolonged in the event that failures occur.

10. A method according to claim 7,
**characterized in that**
the movement of the containers (10) along its transport path (P) on the one hand and the relative movement of the containers (10) relative to their associated sterilization devices (20) on the other hand are decoupled from each other.

## Revendications

1. Installation (1) pour la stérilisation de récipients (10) au moyen de porteurs de charge, comprenant un premier dispositif de transport (2) transportant les récipients (10) le long d'un chemin de transport (P) prédéfini, une pluralité de dispositifs de stérilisation (20) disposée sur ledit dispositif de transport (2), dans laquelle les dispositifs de stérilisation (20) comprenant chacun des dispositifs de génération de porteurs de charge (22) destinés à générer les porteurs de charge, des dispositifs d'accélération (24) destinés à accélérer les porteurs de charge, et des dispositifs de porteurs de charge (26) en forme de barres, lesquels pouvant être introduits dans les récipients (10) par l'embouchure de ceux-ci et dans laquelle les dispositifs de porteurs de charge (26) présentant en outre des fenêtres de sortie (28) par lesquelles les porteurs de charge sortent des dispositifs de porteurs de charge (26),
dans laquelle l'installation (1) comprend au moins un dispositif de détection (30) détectant une défaillance des porteurs de charge accélérés des différents dispositifs de stérilisation (20),
**caractérisée en ce que**
les différents dispositifs de stérilisation (20) comprennent chacun des dispositifs de déplacement (14) destinés à déplacer les récipients (10) dans la direction longitudinale (L) des récipients (10) par rapport aux dispositifs de porteurs de charge (26) en forme de barres,
dans laquelle les profils cinétiques des déplacements des récipients (10) peuvent être commandés indépendamment les uns des autres par rapport aux dispositifs de porteurs de charge (26) qui leur sont respectivement associés,
dans laquelle les dispositifs de déplacement (14) sont en liaison de signaux avec le dispositif de détection (30) de telle manière qu'à la détection par le dispositif de détection (30) d'une défaillance des porteurs de charge accélérés sur un dispositif de stérilisation (20), un déplacement relatif est arrêté et/ou inversé entre ledit dispositif de stérilisation (20) et le récipient (10) à stériliser par ledit dispositif de stérilisation (20), dans laquelle le déplacement est poursuivi après rétablissement de la puissance de rayonnement, dans laquelle le transport des récipients (10) est poursuivi par le dispositif de transport (2) à vitesse constante le long du chemin de transport (P) avec ce déplacement modifié des récipients dans leur direction longitudinale (L) par rapport au dispositif de porteurs de charge (26).

2. Installation (1) selon la revendication 1,
**caractérisée en ce que**
un dispositif de commande (42) commande les dispositifs de stérilisation (20) de telle manière que ceux-ci émettent un rayonnement de porteurs de charge au moins pendant le retrait des dispositifs de porteurs de charge (26) hors des récipients (10), et qu'à la détection d'une défaillance des porteurs de charge accélérés le déplacement relatif du dispositif de porteurs de charge (26) par rapport au récipient (10) soit arrêté, et/ou que le dispositif de porteurs de charge (26) soit réintroduit au moins en partie dans le récipient (10).

3. Installation (1) selon la revendication 1,
**caractérisée en ce que**
l'installation (1) comprend un deuxième dispositif de transport (34) recevant les récipients (10) du premier dispositif de transport (2) à un emplacement de transfert (P2) prédéfini.

4. Installation (1) selon la revendication 3,
**caractérisée en ce que**
un intervalle temporel pendant lequel la fenêtre de sortie se trouve à l'intérieur des récipients (10) est modifiable.

5. Installation (1) selon au moins une des revendications précédentes,
**caractérisée en ce que**
l'installation (1) comporte une salle blanche (40) à l'intérieur de laquelle sont transportés les récipients en matière plastique (10).

6. Installation (1) selon au moins une des revendications précédentes,
**caractérisée en ce que**
le dispositif de transport (2) est un support (2) rotatif.

7. Procédé de stérilisation de récipients (10), dans lequel les récipients (10) étant transportés par un dispositif de transport (2) le long d'un chemin de transport (P) prédéfini et pendant ce transport au moins une paroi intérieure (10a) des récipients (10) étant stérilisée en étant exposée à des porteurs de charge, dans lequel des porteurs de charge étant générés pour la stérilisation des récipients (10) et étant accélérés à l'intérieur d'un dispositif de porteurs de charge (26) en forme de barres, au moyen d'un dispositif d'accélération (24), dans lequel le dispositif de porteurs de charge (26) étant introduit dans le récipient (10) avec un profil cinétique prédéfini, par déplacement relatif entre le dispositif de porteurs de charge (26) et le récipient (10) le long de la direction longitudinale (L) du récipient (10), et avant d'être ressorti du récipient (10), et la paroi intérieure du récipient (10) étant exposée aux porteurs de charge pendant que le dispositif de porteurs de charge (26) se trouve au moins en partie à l'intérieur du récipient (10), dans lequel un dispositif de détection (30) détecte une défaillance temporaire du rayonnement des porteurs de charge,
**caractérisé en ce que**
le dispositif de détection (30), en réaction à une défaillance du rayonnement des porteurs de charge, arrête un déplacement relatif entre le récipient (10) et le dispositif de porteurs de charge et/ou inverse la direction de déplacement, dans lequel le déplacement est poursuivi après rétablissement de la puissance de rayonnement, dans lequel le transport des récipients (10) est poursuivi par le dispositif de transport (2) à vitesse constante le long du chemin de transport (P) avec ce déplacement modifié des récipients dans leur direction longitudinale (L) par rapport au dispositif de porteurs de charge (26).

8. Procédé selon la revendication 7,
**caractérisé en ce que**
une stérilisation des récipients (10) est effectuée pendant que le dispositif de porteurs de charge (26) est retiré des récipients (10).

9. Procédé selon la revendication 7,
**caractérisé en ce que**
l'intervalle temporel pendant lequel les dispositifs de porteurs de charge (26) se trouvent dans les récipients (10) est prolongé en cas de défaillances.

10. Procédé selon la revendication 7,
**caractérisé en ce que**,
d'une par, le déplacement des récipients (10) le long de leur chemin de transports (P), et d'autre part, et le déplacement relatif des récipients (10) par rapport aux dispositifs de stérilisation (20) qui leur sont associés, sont découplés l'un de l'autre.
